Europäisches Patentamt

European Patent Office

Office européen des brevets

(19)

(11) Publication number: **0 104 618**
**A2**

(12) **EUROPEAN PATENT APPLICATION**

(21) Application number: **83109482.6**

(22) Date of filing: **23.09.83**

(51) Int. Cl.³: **A 61 B 10/00**

(30) Priority: **24.09.82 US 422930**

(43) Date of publication of application:
**04.04.84 Bulletin 84/14**

(84) Designated Contracting States:
**AT BE CH DE FR GB IT LI LU NL SE**

(71) Applicant: **Advanced Technology Laboratories, Inc.**
**13208 Northrup Way**
**Bellevue Washington(US)**

(72) Inventor: **Silverstein, Fred Eli**
**2418 E. Louisa**
**Seattle Washington(US)**

(72) Inventor: **Heyerdahl, Norman Edmund**
**15352 158th. N.S.**
**Woodinville Washington(US)**

(74) Representative: **Vossius Vossius Tauchner Heunemann Rauh**
**Siebertstrasse 4 P.O. Box 86 07 67**
**D-8000 München 86(DE)**

(54) **Sterile sheath apparatus for intraoperative ultrasound scanning.**

(57) The apparatus is a sheath which includes a substantially puncture-proof cap and a flexible tubular extension for covering a portion of the cable which extends between the ultrasound scanhead and the ultrasound apparatus. The apparatus is sterilizable to prevent contamination of the patient during procedures, such as a biopsy, which may be conducted under the guidance of ultrasound.

Fig. 1

EP 0 104 618 A2

VOSSIUS · VOSSIUS · TAUCHNER · HEUNEM.

PATENTANWÄLTE

SIEBERTSTRASSE 4 · 8000 MÜNCHEN 86 · PHONE: (089) 47 40 75
CABLE: BENZOLPATENT MÜNCHEN · TELEX 5-29 453 VOPAT D

0104618

23 Sep. 1983

Our Ref.: S 621 EP
Case: V422930-Z

ADVANCES TECHNOLOGY LABORATORIES, INC.
BELLEVUE, WASHINGTON, U.S.A.

WB24

## STERILE SHEATH APPARATUS FOR INTRAOPERATIVE ULTRASOUND SCANNING

The present invention relates to a device used in ultrasound for medical diagnosis. Ultrasound is presently being used during intraoperative procedures to image body structures. Frequently, the ultrasound device must be touched, i.e. in actual physical contact with, an area of the body which is a sterile environment. A typical example is the dura of the brain which would be touched in the course of use of a device such as the Neuro SectOR operating room ultrasound neurology sector scanner made by Advanced Technology Laboratories of Bellevue, Washington.

The surface of most ultrasound devices can be cleaned and disinfected. However, the surfaces of such devices cannot generally be sterilized, because of the inability of the device to undergo soaking and sterilizing solutions or sterilization with gasses, such as ethylene oxide. Therefore, traditional practice for intraoperative ultrasound scanning has been to cover the ultrasound scanhead with a sterile latex sheath which is applied to the device in the operating room environment immediately prior to imaging. Recently, double sheathing has been used to provide increased protection against the possibility of a puncture or cut in the latex cover during surgery. Such punctures can occur from devices used in the course of surgery such as a hemostat, a needle, a scalpel, or, in the case of neurosurgery, a shard of bone from the craniotomy. Accordingly, there is a great deal of concern regarding the use of soft latex in such procedures.

The present invention relates to a sterile sheath for use during surgery which bacteriologically isolates the ultrasound scanhead from the patient or tissue surface. In accordance with the present invention, a sterile sheath for an ultrasound scanhead includes a proximal tube which extends for a sufficient distance, typically two to three feet, up the electronic cable from the scanhead device. The proximal tube is preferably made of a flexible, sterile, waterproof material. As the proximal tube does not have to be especially resistant to puncture, it may be comprised of a resilient material, such as latex. The sterile sheath further comprises a cap which goes over the scanhead itself. The cap is constructed of a strong, durable material which will directly resist cutting and puncturing during operative procedures. The cap is comprised of a rigid, sterilizable material which permits sufficient acoustical power transmission to allow for effective imaging of the target organ. The cap and the proximal tube are joined together by a continuous, sterile, waterproof seal, so the entire system from the proximal, flexible tube to the durable, rigid cap is water tight and sterile.

FIG. 1 illustrates a first embodiment of the present invention;

FIG. 2 illustrates a second embodiment of the present invention; and

FIG. 3 illustrates a the embodiment of FIG. 2 with the addition of needle guides.

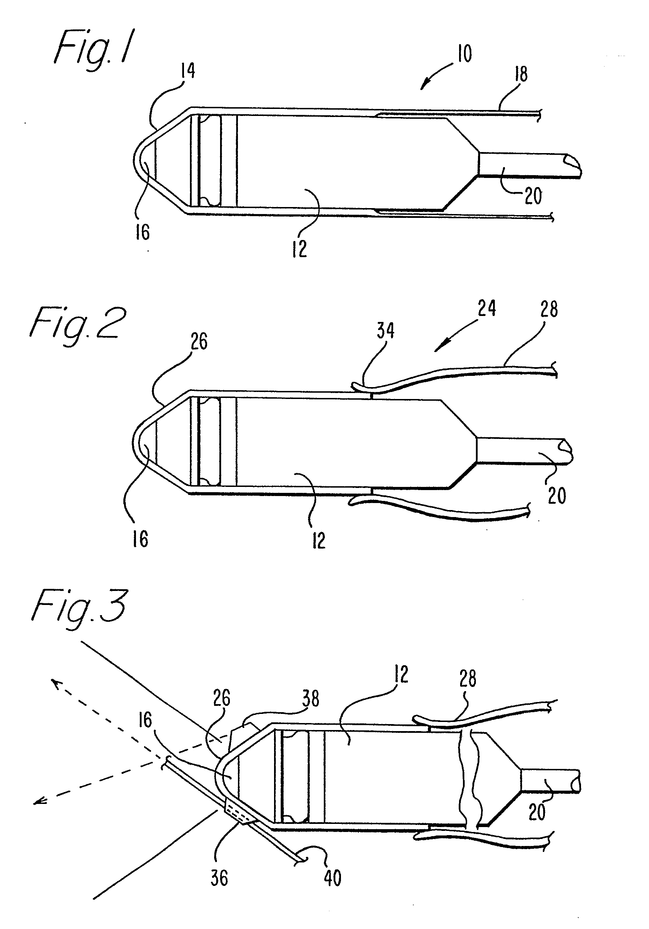

Referring generally to FIG. 1, a sterile sheath apparatus 10 is illustrated in cross-section over an ultrasound scanhead 12. The sheath 10 is comprised of two portions, namely a first cap portion 14 which extends over the end 16 of the ultrasound scanhead 12 and a second tubular portion 18 which extends back over the cable 20 from the ultrasound scanhead 12. In the present embodiment, the entire sheath 10 is made up of a single material. However, the sheath 10 has two distinct portions 14, 18. Thus, the portion 14 of the sheath 10 which extends over the ultrasound scanhead 12 is made to be extremely thick, so it is highly unlikely that that portion 14 would be punctured during an operative procedure. On the other hand, the tubular portion 18, which extends back over the cable 20, is relatively thin-walled, similar to the latex sheaths heretofore used, in order to provide flexibility while retaining the sterility and waterproof qualities heretofore required. In the present embodiment of the invention, the entire sheath 10 can be constructed of latex or polyethylene.

Referring now to FIG. 2, a second embodiment of the invention is shown. In the second embodiment the sheath 24 is comprised of two parts. The first part is a cap 26 made of a rigid material designed to fit over the end 16 of a scanhead 12. The second portion of the sheath 24 is the flexible, tubular portion 28 which extends back over the cable 20 from the scanhead 12. The cap 26 can be comprised of any inexpensive, sterilizable material, such as a plastic. In particular, it may be made of polyethylene. The proximal tube 28, which which is preferably made of an inexpensive, sterilizable material, such as a sterilizable plastic or latex, can be firmly attached to the cap 26 prior to sterilization by a shrink process, by an adhesive, by a clamp, or by any other method which provides for a sterile, continuous, waterproof seal 34.

Referring now to FIG. 3, a particular feature of the present invention, particularly the version of the invention shown in FIG. 2, is the ability to build guide channels 36, 38 into the wall of the cap 26. The guide channels 36, 38 can be used to guide a variety of devices into a patient under while their placement is monitored using the ultrasound scan. The channels 36, 38 can be used to direct the placement of a needle 40 into a target tissue while keeping the needle 40 in the plane of the sector scanner 12. An additional guide 38 could have a different insertion angle on the opposite side of the cap 26, as shown. Thus, a first channel 36 on one side of the cap 26 could provide an entry at one angle into the target tissue within the scanned sector while a second channel 38 on the opposite side of the cap 26 could provide a second entry angle into the sector being scanned. Other devices, such as an outboard directional doppler device (not shown), could also be placed into a channel whereby it would be possible to provide for simultaneous doppler and two-dimentional imaging.

While the present invention has been described with particular reference to a mechanical ultrasound sector scanner, those of ordinary skill in the art will recognize that it may be used, also, with either a phased array, an annular array or a linear scanner in particular applications.

We Claim:

1.  A sterile sheath apparatus for intraoperative ultrasound scanning comprising:

(a)  a first substantially puncture-proof cap portion designed to fit over the portion of an ultrasound scanning unit which contacts a patient, said cap being made of a material which is substantially transparent to ultrasound and which is sterilizable; and

(b)  a second tubular portion which is joined to said cap and which extends back from said cap to cover the cable connecting said ultrasound scanner to the display apparatus.

2.  The sterile sheath apparatus for intraoperative ultrasound scanning of Claim 1 wherein said ultrasound scanner is a mechanical sector scanner and said cap is fitted over the end of said sector scanner.

3.  The sterile sheath apparatus for intraoperative ultrasound scanning of Claim 2 wherein said cap and said tubular portion are made of the same material, and said cap is substantially thicker than said tubular portion.

4.  The sterile sheath apparatus for intraoperative ultrasound scanning of Claim 3 wherein said cap and said tubular portion are made of latex.

5. The sterile sheath apparatus for intraoperative ultrasound scanning of Claim 2 wherein said cap and said tubular portion are made of different materials which are joined together by a sterile, waterproof seal.

6. The sterile sheath apparatus for intraoperative ultrasound scanning of Claim 5 wherein said cap is made of a sterilizable plastic.

7. The sterile sheath apparatus for intraoperative ultrasound scanning of Claim 6 wherein said tubular portion is made of latex.

8. The sterile sheath apparatus for intraoperative ultrasound scanning of Claim 7 wherein said cap is made of polyethylene.

*Fig.1*

*Fig.2*

*Fig.3*